# EUROPÄISCHE PATENTSCHRIFT

(11) **EP 0 981 297 B1**
(45) Veröffentlichungstag und Bekanntmachung des Hinweises auf die Patenterteilung: **11.08.2004**
(21) Anmeldenummer: 98922724.4
(22) Anmeldetag: 16.04.1998
(51) Int. Cl.: A61B 17/11, A61B 17/02

(54) **ANORDNUNG ZUM LOKALEN RUHIGSTELLEN EINES SCHLAGENDEN HERZENS**
DEVICE FOR LOCALLY IMMOBILIZING A BEATING HEART
SYSTEME D'IMMOBILISATION LOCALE D'UN COEUR EN TRAIN DE BATTRE

(30) Priorität: 07.05.1997 DE 29708050 U
(43) Veröffentlichungstag der Anmeldung: 01.03.2000
(73) Patentinhaber: Riess, Andreas G., 22850 Norderstedt (DE)
(72) Erfinder: Riess, Andreas G., 22850 Norderstedt (DE)
(74) Vertreter: Böhme, Ulrich, Dr. Dipl.-Phys.
(86) Internationale Anmeldenummer: PCT/EP1998/002234
(87) Internationale Veröffentlichungsnummer: WO 1998/049947

(56) Entgegenhaltungen:
- EP-A- 0 791 330
- WO-A-94/04080
- WO-A-95/17919
- WO-A-97/10753
- WO-A-97/40738
- DE-U- 29 707 567
- US-A- 5 447 515

## Beschreibung

Die Erfindung betrifft eine Anordnung zum lokalen Ruhigstellen eines schlagenden Herzens, insbesondere zum Zweck der Herstellung einer Anastomose zwischen einem Bypassconduit und einem Koronargefäß der Vorderwand der linken Herzkammer, mit einer Plattform, die aus mindestens zwei Plattformteilstücken besteht, welche so miteinander verbunden sind, daß sich die Plattformteilstücke so gegeneinander bewegen lassen, daß die Plattformteilstücke sich teilweise oder ganz voneinander lösen.

Minimal invasive Operationstechniken liegen im Trend und erfahren gerade heute vor dem Hintergrund der Notwendigkeit zur Kosteneinsparung reges Interesse. Dabei geht es jedoch nicht ausschließlich um die Kosten. Auch die Verringerung des prä-, intra- und postoperativen Traumas, die verkürzten Operations- und Narkosezeiten, die schnellere Wundheilung, kürzere Liegezeiten und weniger Wundschmerz sowie das kosmetische Ergebnis sind wichtige Argumente für minimal invasive Operationstechniken. In allen Bereichen der Chirurgie gibt es Bestrebungen, die Standardprozeduren durch minimal invasive Varianten zu ersetzen. Beispielsweise sei nur auf die videokontrollierte Trokar-Technik für gynäkologische Eingriffe verwiesen.

Herzchirurgischen Operationen mit ihrer gemessen an der Größe des Eingriffes geringen Mortalität und Morbidität wurden erst durch die Entwicklung der Herz-Lungen-Maschine in den 50er Jahren möglich. Diese erlaubt für einen Zeitraum von mehreren Stunden Operationen an einem ruhiggestellten und blutleeren Herzen vorzunehmen. Für mehrere Jahrzehnte waren Operationen am Herzen mit der Herz-Lungen-Maschine der Goldstandard.

In einer wesentlichen Fortentwicklung wurden später bestimmte Koronaroperationen in minimal invasiven Operationstechniken am schlagenden Herzen vorgenommen, wobei der Einsatz der Herz-Lungen-Maschine überflüssig wurde. Die Motivation für dieses Vorgehen war das zunehmende Wissen um die Nebenwirkungen und Nachteile des kardiopulmonalen Bypass (CPB). Der Kontakt des Blutes mit Kunststoffoberflächen der Herz-Lungen-Maschine führt zu einer Aktivierung der sogenannten Gerinnungskaskade und des Komplementsystems. Um zu verhindern, daß es dadurch bedingt zu einer Blutgerinnselbildung in der Herz-Lungen-Maschine und dadurch zum Verschluß derselben kommt, sind hohe Dosen an Heparin notwendig. Durch die dadurch bewirkte komplette Aufhebung der Blutgerinnung kann es während und nach Eingriffen mit Herz-Lungen-Maschine zu Blutungskomplikationen kommen. Dadurch kann die Verabreichung von Fremdblut mit all ihren möglichen Konsequenzen (Hepatitis, HIV, u.a.) notwendig werden. Auch die Blutplättchen, welche für eine normale Blutgerinnung essentiell sind, werden durch einen Eingriff mit Herz-Lungen-Maschine zum Teil erheblich in ihrer Funktion beeinträchtigt, welches wiederum das Risiko für Blutungskomplikationen ansteigen läßt.

So verwundert es nicht, daß bei randomisierten prospektiven Studien mit größeren Anzahlen von Patienten ein Ergebnis war, daß die Patienten, welche am schlagenden Herzen ohne Herz-Lungen-Maschine operiert worden waren, postoperativ einen statistisch signifikant geringeren Blutverlust hatten, als die Patienten, welche mit Herz-Lungen-Maschine operiert wurden. Die Tatsache, daß bei Eingriffen mit Herz-Lungen-Maschine in der Regel das gesamte Brustbein längs durchtrennt werden muß, kann zu postoperativen Schmerzen im Wundbereich, aber auch zu Wundheilungsstörungen und Instabilität des Brustbeines führen. Weitere Nebenwirkungen von Eingriffen mit Herz-Lungen-Maschine sind neurologische Komplikationen aus der Herz-Lungen-Maschine, die in Zusammenhang mit der extrakorporalen Zirkulation gebracht werden. So können kleine Mikrogerinnsel, aber auch Luftembolien in Hirnarterien gelangen und dort Schlaganfälle auslösen. Eine weitere Quelle von thromboembolischen Komplikationen können feine Verkalkungen im Bereich der Hauptschlagader sein, die durch die Manipulationen an derselben (Anschluß der Herz-Lungen-Maschine und Abklemmen bzw. seitliche Ausklemmung der Hauptschlagader) abgesprengt werden können. Außerdem ist es bekannt, daß nicht wenige Patienten nach einem Eingriff mit Herz-Lungen-Maschine und kardioplegischem Herzstillstand geringgradige neurologische Ausfälle bzw. psychiatrische Auffälligkeiten (bis zu 30 Prozent) aufweisen können.

Dem gegenüber bietet die minimal invasive Versorgung des wichtigsten Gefäßes der linksventrikulären Vorderwand (LAD) ohne Herz-Lungen-Maschine zahlreiche Vorteile. Die Operation ist bei einem geübten Chirurgen schneller durchzuführen als ein Eingriff mit Herz-Lungen-Maschine. Die Patienten haben eine kleinere Narbe und somit wird ein kosmetisch besseres Ergebnis erzielt. Das Brustbein behält einen Teil seiner Stabilität, da es nur partiell durchtrennt wird. Dies bedingt weniger Wundschmerzen und ermöglicht eine in der Regel unkomplizierte Verheilungsphase des Knochens. Bei der LIMA/LAD (Brustbeinschlagader / Koronargefäß) Prozedur wird das wichtigste Gefäß des Herzens (LAD) mit dem besten Bypassconduit (LIMA) versorgt. Bis zu 80 Prozent des gesamten Blutbedarfs des Herzens kann durch den LAD gedeckt werden. Die meisten Patienten sind nach einer LIMA/LAD Prozedur, auch wenn weitere Stenosen in kleineren Ästen bestehen, nach erfolgter Operation beschwerdefrei, auch wenn die anderen Stenosen unbehandelt bleiben. Dennoch sollten diese Stenosen, sofern sie denn vorhanden sind, nach erfolgter minimal invasiver LIMA/LAD Prozedur aus prognostischer Sicht mit einem dann geringeren Risiko dilatiert werden, da zuvor der LAD versorgt worden ist. Betrachtet man die Offenheitsraten der verschiedenen Bypasstypen auf die verschiedenen HerzgefäBe, so wird das Obengesagte nochmals verdeutlicht. Die Brustbeinschlagaderversorgung des LAD hat eine 10-Jahres-Offenheitsrate von 93 Prozent. Dem gegenüber können die Venenbypypässe schon nach wenigen Jahren Veränderungen der Gefäßinnenwand zeigen, und die Offenheitsrate von Venenbypässen liegt, je nachdem auf welches Gefäß sie genäht wurden, nach zehn Jahren nur zwischen 40 bis 80 Prozent.

Weitere Vorteile bei der minimal invasiven Koronar-Chirurgie sind die kurzen Narkosezeiten, eine in der Regel auf dem Operationstisch erfolgende Extubation, der nur wenige Stunden dauernde Aufenthalt auf der Intensivstation und ein Gesamthospitalaufenthalt von ca. zwei bis vier Tagen. Dies ist für den Patienten vorteilhaft und Kosten können reduziert werden. Ferner kommt es bedingt durch das kleinere wundgebiet zu geringeren Verwachsungen zwischen Herzbeutel und Herz, was für eventuelle spätere Re-Operationen von Wichtigkeit sein kann. Außerdem wird in Studien berichtet, daß das Auftreten von Herzrhythmusstörungen im postoperativen Zeitraum nach minimal invasiven herzchirurgischen Eingriffen geringen ist.

Zur Herstellung der Anastomose zwischen LIMA und LAD muß am schlagenden Herz der Anastomosenbereich ruhiggestellt werden, um die ca. 15 Stiche in einem Bereich von wenigen Millimetern mit der erforderlichen Präzision vornehmen zu können.

Die LIMA/LAD Prozedur am schlagenden Herzen ist bekannt und wird praktiziert. Hierbei wird am beatmeten Patienten eine sogenannte Mini-Sternotomie vorgenommen. Es wird eine ca. acht Zentimeter lange Hautinzision beginnend ca. zwei Zentimeter oberhalb des Schwertfortsatzes bis auf Höhe des vierten Interkostalraumes (ICR) durchgeführt. Anschließend wird eine partielle mediane Sternotomie bis in den linken dritten ICR vorgenommen. Die LIMA wird unter direkter Sicht des Auges bis ca. zum zweiten ICR präpariert. Anschließend wird die Gerinnungszeit des Blutes verlängert, indem 5000 bis 7500 Einheiten Heparin intravenös verabreicht werden. Anschließend wird distal und proximal des für die Anastomose gewählten Bereiches der LAD angeschlungen und dadurch occludiert. Bei der anschließend durchzuführenden End-zu-Seit-Anastomose zwischen LIMA und LAD mit einer fortlaufenden 8-0 Naht ist es für die Qualität der Anastomose und damit für den Erfolg der Operation insgesamt von größter Bedeutung, wie gut es gelingt, den Anastomosenbereich zu stabilisieren.

Es wurden dafür verschiedene Werkzeuge entwickelt, die zum Teil von einem Assistenten zu halten sind oder auf verschiedene Weise festgesetzt werden. Diese Werkzeuge sind entweder unter hohem Druck auf den Operationsbereich aufzupressen oder besitzen Saugnäpfe, mit deren Hilfe das Herz angehoben wird. In beiden Fällen ist es jedoch besonders nachteilig, daß es durch die starken Druck- oder Saugkräfte zu Einrissen oder Hämatomen am Herzgewebe kommen kann.

Eine Anordnung zum lokalen Ruhigstellen eines schlagenden Herzens der eingangs genannten Art ist beispielsweise aus der WO 97/10753 bekannt. Bei dieser bekannten Vorrichtung können Plattformteilstücke mit einem bestimmten Abstand voneinander am Herzen angebracht werden, die gegeneinandergefahren werden können und die als Saugflächen ausgebildet sind. Auch hier kann es durch die Saugwirkung zu Verletzungen kommen.

Es ist die Aufgabe der vorliegenden Erfindung, eine Anordnung zum lokalen Ruhigstellen eines schlagenden Herzens so fortzubilden, daß der Anastomosenbereich ruhiggestellt werden kann, ohne daß es durch starke Druckkräfte zu Einrissen oder zu Hämatomen am Herzgewebe kommen kann.

Diese Aufgabe wird bei einer Anordnung der eingangs beschriebenen Art erfindungsgemäß dadurch gelöst, daß die Plattform einen Durchbruch aufweist, dessen Saum anteilig von beiden Plattformteilstücken gebildet wird.

Der Anastomosenbereich wird durch das Heranzügeln des occludierten LAD an den Durchbruch der Plattform fixiert. Jenseits des Durchbruchrandes kann das Herz ungehindert seine Expansions- bzw. Kontraktionsbewegungen ausfahren. Dadurch kann der Anastomosenbereich in allen Richtungen fixiert werden.

Dies hat den Vorteil, daß bei verbesserter Stabilisierung die Traumatisierung des Herzgewebes weiter minimiert wird.

Der Durchbruch ist vorzugsweise ovalartig und in den Bereichen, in denen der LAD in den Durchbruchbereich hinein- bzw. hinausläuft, tunnelartig gewölbt, um eine mögliche Verletzung des LAD durch Kanten zu verhindern. Der ovalartige Durchbruch hat einen kleinen Durchmesser von 7 mm bis 16 mm, insbesondere von 10 mm, und einen großen Durchmesser von 9 mm bis 20 mm, insbesondere von 14 mm.

Auf jedem der beiden Plattformteilstücke ist jeweils mindestens eine Öffnung vorgesehen, wobei ferner Mittel zum Umschlingen des Koronargefäßes vorgesehen sind, welche durch die wenigstens zwei Öffnungen hindurchführbar und an wenigstens einer auf den Plattformteilstücken vorgesehenen Befestigungsvorrichtungen befestigbar sind. Die Idee ist dabei im wesentlichen, auf das Niederdrücken zum Zweck der Stabilisierung ganz zu verzichten. Vielmehr wird die Plattform auf den LAD gesenkt und dieser zum Zweck der Arretierung im Bereich des ovalartigen Durchbruches angezügelt. Das Konzept zielt darauf, die Friktion so gering wie möglich zu halten und dadurch das Herz so wenig wie möglich in seiner Bewegung zu behindern. Dabei ist die Fläche der Plattform vergleichsweise groß und auf der Unterseite im wesentlichen eben und glatt. Der Kernpunkt ist, daß die Ruhigstellung des LAD einzig durch die Anzügelung, und damit das Hereinziehen des Ananstomosenbereiches in den ovalartigen Durchbruch der Plattform bewerkstelligt wird. Friktionskräfte sind für diese Form der Fixierung nicht erforderlich.

Dies hat den Vorteil, daß bei verbesserter Stabilisierung und für den Operateur optimaler Zugänglichkeit des zu operierenden Koronargefäßes gleichzeitig die Traumatisierung des Herzgewebes minimiert wird.

Nach erfolgtem Aufnähen der LIMA auf den LAID ist die Plattform von der durch den ovalartigen Durchbruch fahrenden LIMA gefangen. Um die Plattform entfernen zu können, kommt nun der Vorteil zum tragen, daß bei der erfindungsgemäßen Anordnung die Plattform aus zwei Teilstücken besteht, welche gegeneinander beweglich angeordnet sind. In einer bevorzugten Ausführung bildet der Stiel der Plattform zusammen in einer im Stiel verlaufenden Welle ein Scharnier, an welchem die Plattformteilstücke so angebracht sind, daß sich die Plattformteilstücke zangenartig öffnen und schließen lassen. Ein Schneckentrieb im Kopf des Plattformstieles dient dabei zur Rotation der Welle in Bezug auf den Plattformstiel. Nachdem die Anastomose im geschlossenen Zustand durchgeführt wurde, werden die Plattformteilstükke geöffnet, wodurch die LIMA freigegeben wird und die Plattform aus dem Operationsfeld entfernt werden kann.

Ein weiterer Vorteil der erfindungsgemäßen Anordnung liegt in folgendem:

Es wird eine Mini-Sternotomie mit einer Länge von beispielsweise 8 bis 10 cm ermöglicht. Dies vermeidet die asymmetrische Öffnung unter Durchtrennung der Muskulatur sowie des Gefäß/Nervenbündels des ICR. Dadurch werden die zum Teil erheblichen Wundschmerzen einer lateralen Thorakotomie vermieden, die oft die Verabreichung von Opiaten über einen längeren Zeitraum nötig machen. Im Vergleich dazu treten bei der medianen Sternotomie und insbesondere bei der Mini-Sternotomie erstaunlich geringfügige postoperative Schmerzen auf, so daß der postoperative Analgetikabedarf gering ist.

Es erfolgt eine maximale Ruhigstellung des Anastomosenbereiches bei gleichzeitig optimaler Justierbarkeit, minimaler Beeinträchtigung der Herzfunktion durch die Anordnung und minimaler Traumatisierung des Herzens durch den Kontakt mit der erfindungsgemäßen Anordnung. Der Operateur hat eine freie Sicht auf das Operationsfeld und die Herzbewegung ist visuell ausgeblendet. In der operativen Praxis hat es sich gezeigt, daß die Aktion des schlagenden Herzens eine visuelle Beeinträchtigung des Operationsfeldes darstellt, welche die Konzentration auf den vergleichsweise kleinen Anastomosenbereich erschwert.

Die Möglichkeit der Fixierung des LAD an den Plattformteilstücken mit entsprechenden Mitteln bringt eine erheblich höhere Anwendungssicherheit und eine verbesserte Ruhigstellung des Operationsbereiches ohne die Notwendigkeit von Anpressdruck auf die Herzmuskulatur.

Die Anordnung ist schnell und einfach montierbar und sekundenschnell demontierbar. Wenn es bei der Occlusion des LAD zu stärkeren hämodynamischen Beeinträchtigungen der Pumpfunktion des Herzens bzw. zu malignen Herzrhythmusstörungen kommt, kann es erforderlich werden, die Operationsstrategie zu wechseln, und die mediane Sternotomie zu komplettieren und mit Hilfe der Herz-Lungen-Maschine die Operation fortzusetzen. Um den Zeitraum einer hämodynamischen Beeinträchtigung und damit die Phase einer Sauerstoffmangelsituation des Gehirns möglichst kurz zu halten, ist es erforderlich, daß eine eingebrachte Stabilisierungsplattform sekundenschnell demontiert werden kann.

Ist die Anastomose fertiggestellt, so werden die beiden Plattformteilstücke geöffnet und die Mittel zur Umschlingung gelöst, worauf sich die Plattform ohne weiteres aus der Operationshöhle herausnehmen läßt.

Die Anordnung ist ferner wiederverwendbar. Im Rahmen der Kostendämpfung im Gesundheitssystem und dem zunehmenden Problem der Entsorgung erscheinen Einmalartikel nicht mehr zeitgemäß. Robustheit und Langlebigkeit für eine beliebig häufige Wiederverwendbarkeit durch Resterilisierbarkeit ist daher einer der wesentlichen Vorteile der erfindungsgemäßen Anordnung.

Durch die vergleichsweise geringe Auflagefläche bisheriger Lösungen kommt es während des Drückens auf den Herzmuskel an den umlaufenden Rändern zu mehr oder weniger starken Knickungen und damit zur Traumatisierung des Herzmuskels. Erfindungsgemäß ist die Fläche der Plattform so groß bemessen, daß es nicht zum Knicken des Herzmuskels an den umlaufenden Rändern kommen kann. Vielmehr wird der LAD mit seinem Begleitgewebe nur vom Rand des ovalartigen Durchbruches gehalten. Die Fläche der Plattform beträgt beispielsweise 10 bis 30 Quadrat-Zentimeter, bei einer bevorzugten Ausführungsform 15 Quadratzentimeter.

Vorzugsweise Weitergestaltungen des Apparats sind in den Ansprüchen 2 bis 12 beschrieben.

In besonders vorteilhafter Weise ist das Befestigungsmittel wenigstens ein Profilknopf mit pilzartigem Querschnitt. Dies erlaubt ein einfaches und schnelles Belegen der Mittel zum Umschlingen des Koronargefäßes an der erfindungsgemäßen Anordnung.

Dadurch, daß die wenigsten zwei Öffnungen je auf einem Plattformteilstück ausgebildet sind, kann ein zu operierendes Gefäß mit dem Mittel zum Umschlingen des Gefäßes im Bereich des ovalartigen Durchbruches einspannen und somit wirksam stabilisieren bzw. ruhigstellen.

Zweckmäßigerweise sind die wenigstens zwei Öffnungen Bohrungen in den Plattformteilstücken.

In einer bevorzugten Ausführungsform wird das Ein- und Ausfädeln der Mittel zum Umschlingen des Koronargefäßes durch die Öffnungen in den Plattformteilstücken dadurch vermieden, daß in den Plattformteilstücken Schlitze vorgesehen sind, welche ausgehend von den Kanten, an denen sich die Plattformteilstücke berühren, zu je einer Öffnung verlaufen. Zweckmäßigerweise sind dabei die Schlitze jeweils haken- oder S-förmig ausgebildet, so daß ein unbeabsichtigtes Herausrutschen der Mittel zum Umschlingen des Koronargefäßes aus den Öffnungen im geöffneten Zustand der Plattform verhindert ist.

Eine besonders schonende und sichere Auflage der Anordnung am Herzen wird dadurch erzielt, daß wenigstens ein Plattformteilstück an einer am Herzen aufliegenden Seite zum ovalartigen Durchbruch hin angefast ist. Der Anfaswinkel beträgt vorzugsweise 5 bis 15 Grad, insbesondere 10 Grad. Zusätzlich ist der Saum des Durchbruches entschärft.

Zweckmäßigerweise ist das Mittel zum Umschlingen des Koronargefäßes wenigsten ein Faden oder wenigsten ein Vesselloop (hohler Gummizügel).

Nachstehend wird die Erfindung anhand der beigefügten Zeichnungen näher erläutert. Diese zeigen in
- Fig. 1: eine erfindungsgemäße Anordnung in perspektivischer Explositionsdarstellung,
- Fig. 2: eine erfindungsgemäße Anordnung mit Feststellmitteln in perspektivischer Ansicht,
- Fig. 3: eine Plattform in geschlossenem Zustand in perspektivischer Ansicht,
- Fig. 4: eine Plattform in geöffnetem Zustand in perspektivischer Ansicht,
- Fig. 5: ein Auslegergelenk in Explositionsdarstellung,
- Fig. 6: eine während einer Operation montierte Anordnung in perspektivischer Ansicht,
- Fig. 7: eine erfindungsgemäße Anordnung mit Feststellmitteln in einer weiteren perspektivischen Ansicht,
- Fig. 8: eine Befestigungsvorrichtung in Schnittansicht,
- Fig. 9: eine vergrößerte Teilansicht einer Plattform in perspektivischer Darstellung,
- Fig. 10: eine zweite Ausführungsform einer erfindungsgemäßen Anordnung,
- Fig. 11 und 12: eine Illustration der Fixierung eines Koronargefäßes an einer erfindungsgemäßen Anordnung in perspektivischer Ansicht,
- Fig. 13: eine vergrößerte Darstellung eines in der erfindungsgemäßen Anordnung fixierten Gefäßes,
- Fig. 14: eine bevorzugte Ausführungsform einer Plattform,
- Fig. 15: eine weitere bevorzugte Ausführungsform einer Plattform,
- Fig. 16A bis 16D: eine Schnittansicht verschiedener Herzzustände bei einem in der erfindungsgemäßen Anordnung fixiertem Koronargefäß und
- Fig. 17: eine dritte Ausführungsform einer erfindungsgemäßen Anordnung.

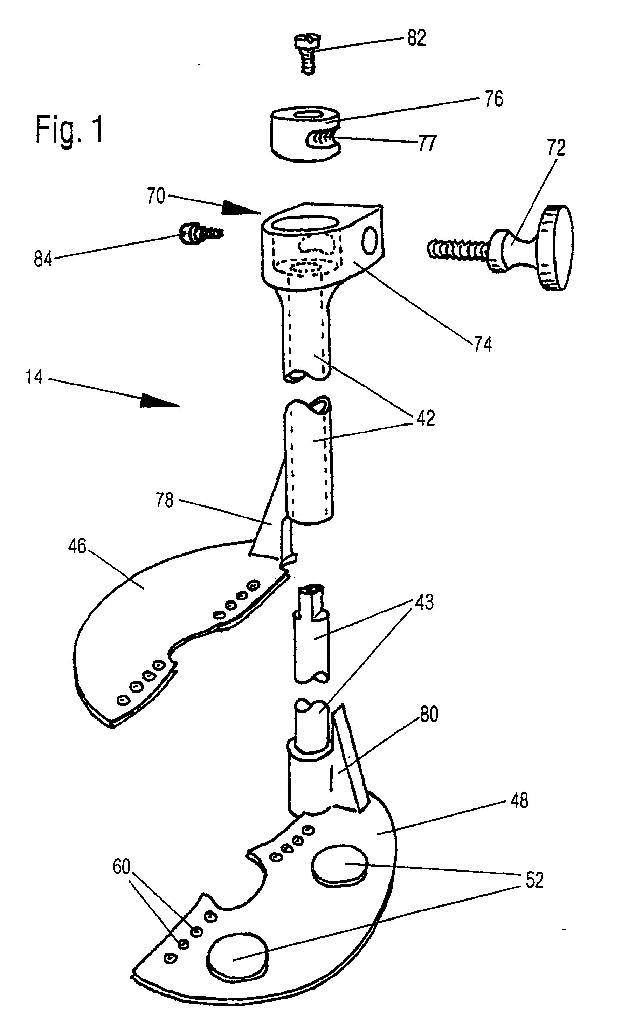

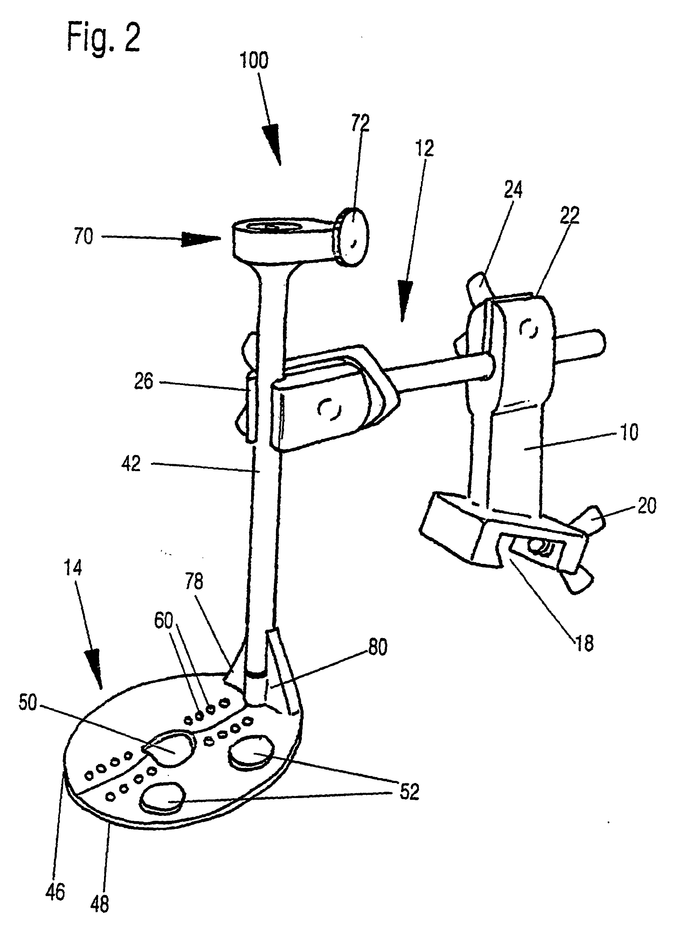

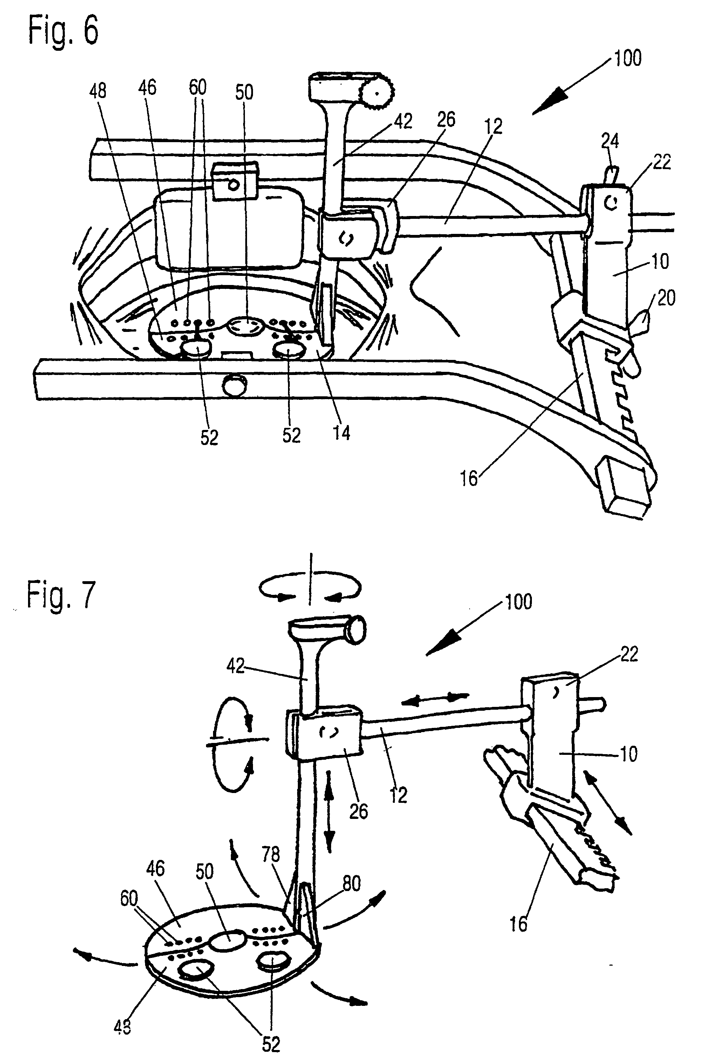

Die in Fig. 1, 2, 6 und 7 dargestellte erfindungsgemäße Anordnung 100 umfaßt im wesentlichen drei Teile: Eine Säule 10, einen Ausleger 12 und eine Plattform 14. Die Säule gleitet mit einem Anschluß 16, z.B. auf der Basis eines BIRNBAUM-Sperrers und läßt sich durch Anziehen einer Flügelschraube 20 in jeder erreichbaren Position festsetzen. Im 90 Grad-Winkel trägt sie den Ausleger 12 in einem Gelenk 22, welches es erlaubt, den Ausleger 12 vor- und zurückzuschieben, sowie ihn um seine Längsachse um jeden beliebigen Winkel zu drehen (vgl. Fig. 7). Das Gelenk 22 läßt sich durch Anziehen einer Flügelmutter 24 festsetzen.

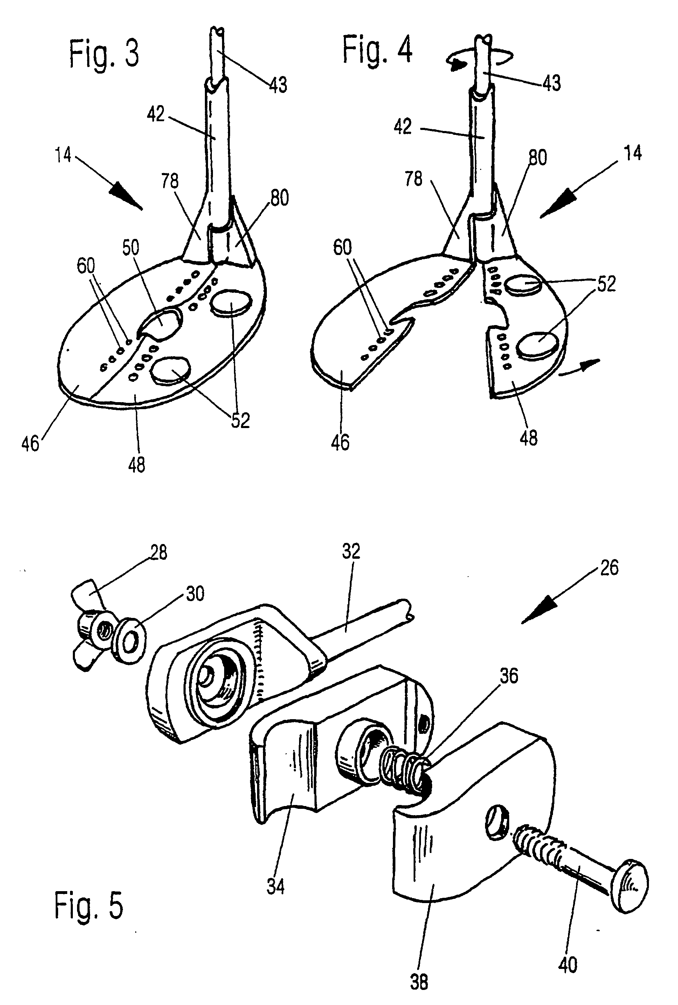

Am Ausleger 12 ist an einem Ende ein weiteres Gelenk 26 angeordnet. Dieses ist in Fig. 5 im Detail dargestellt und umfaßt eine Flügelmutter 28, eine Unterlegscheibe 30, einen Auslegerstiel 32, eine innere Schale 34, eine Druckfeder 36, eine äußere Schale 38 und einen Schraubenbolzen 40, beispielsweise einen M6-Bolzen.

Die Freiheitsgrade von Säule 10 und Stiel 12 bzw. 32 erlauben es, dieses Gelenk 26 auf einer festen Ebene über dem Operationsfeld beliebig zu positionieren und in einem beliebigen Raumwinkel auszurichten (vgl. Fig. 7). Weiter ist dieses Gelenk 26 so beschaffen, daß es einen Stiel 42 der Plattform 14 aufnehmen kann und dieser in Sekundenschnelle durch Anziehen der Flügelmutter 28 festsetzbar ist.

Die Plattform 14 umfaßt den vorgenannten Stiel 42, innerhalb dessen die Welle 43 verläuft, den Plattformkopf 70, sowie die Plattformteilstücke 46 und 48.

Das Plattformteilstück 46 ist über den Winkel 78 mit dem unteren Ende des Stiels 42 verbunden, das Plattformteilstück 48 ist über den Winkel 8C mit dem unteren Ende der Welle 43 verbunden. Bei eingeschobener Welle 43 in den Stiel 42 bilden die Welle 43, der Stiel 42 zusammen mit den Winkeln 78 und 80 ein Scharnier, welches es erlaubt, die an der Basen der Winkel befestigten Plattformteilstücke zangenartig zu öffnen und zu schließen.

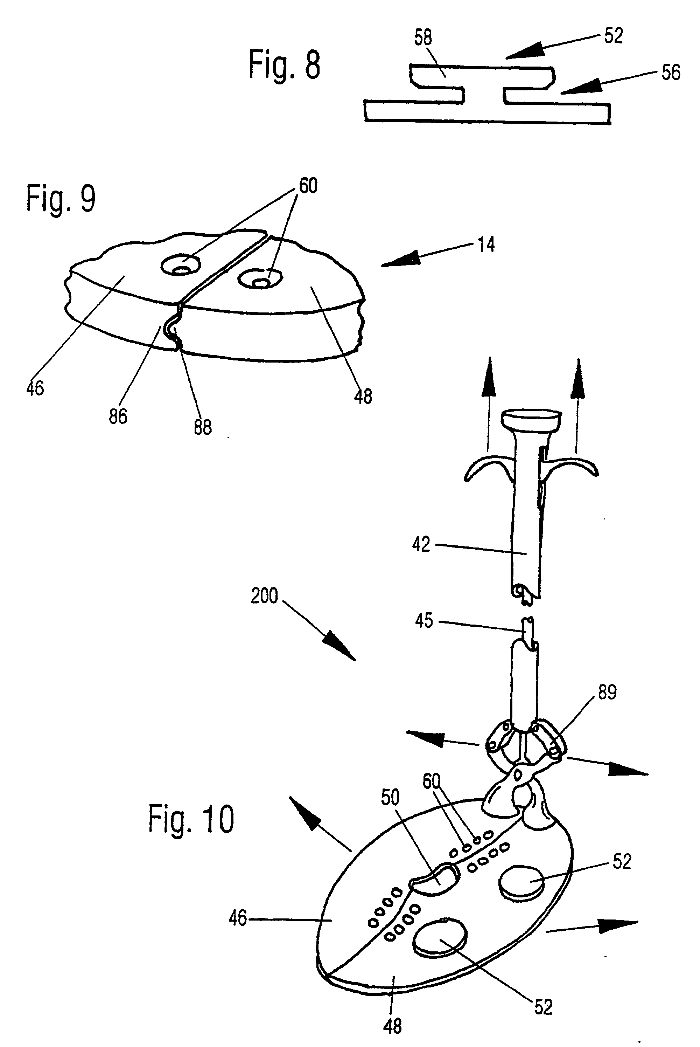

Die Innenkanten der Plattformteilstücke 46 und 48 weisen ein Nut- bzw. Federprofil (86, 88) auf, wodurch im geschlossenen Zustand der Plattformteilstücke 46 und 48 eine Verzahnung erreicht wird (Fig. 9).

Am oberen Ende des Stiels 42 befindet sich der Plattformkopf 70, bestehend aus Gehäuse 74, Schneckenrad 76, Rändelschraube 72, sowie aus Schrauben 82 und 84. Das Gehäuse 74 ist fest mit dem Stiel 42 verbunden.

Auf das obere Ende der Welle 43 wird das Schneckenrad 76 mit der Schraube 82 befestigt. Durch geeignete Formgebung des Wellenendes und der entsprechenden Aufnahme für das Wellenende im Schneckenrad 76 wird bewerkstelligt, daß die Verbindung zwischen Schneckenrad 76 und Welle 43 verdrehsicher ist. Das Schneckenrad 76 sitzt paßgenau und um die Achse der Welle 43 drehbar im Gehäuse 74. Das mit der Schraube 82 auf die Welle 43 geschraubte Schneckenrad hält die Welle 43 und damit das Plattformteilstück 48 in genau der Höhe, daß beim Schließen der Plattformteilstücke 46 und 48 diese sich unter paßgenauer Verzahnung zu einer Fläche vereinigen.

Zum Öffnen und Schließen der Plattformteilstücke 46 und 48 ist die Rändelschraube 72 so im Gehäuse angeordnet, daß sie mit ihrem Gewinde den teilweise oder ganz um das Schneckenrad 76 laufenden Zahnkranz 77 tangential erfaßt. Die Rändelschraube 72 ist durch die Schraube 84 in Achsenrichtung im Gehäuse 74 fixiert. Das Drehen der Rändelschraube erzeugt so ein Drehmoment auf das Schneckenrad 76, welches über die Welle 43 und den Winkel 80 das Plattformteilstück 48 bezüglich der Wellenachse schwenkt.

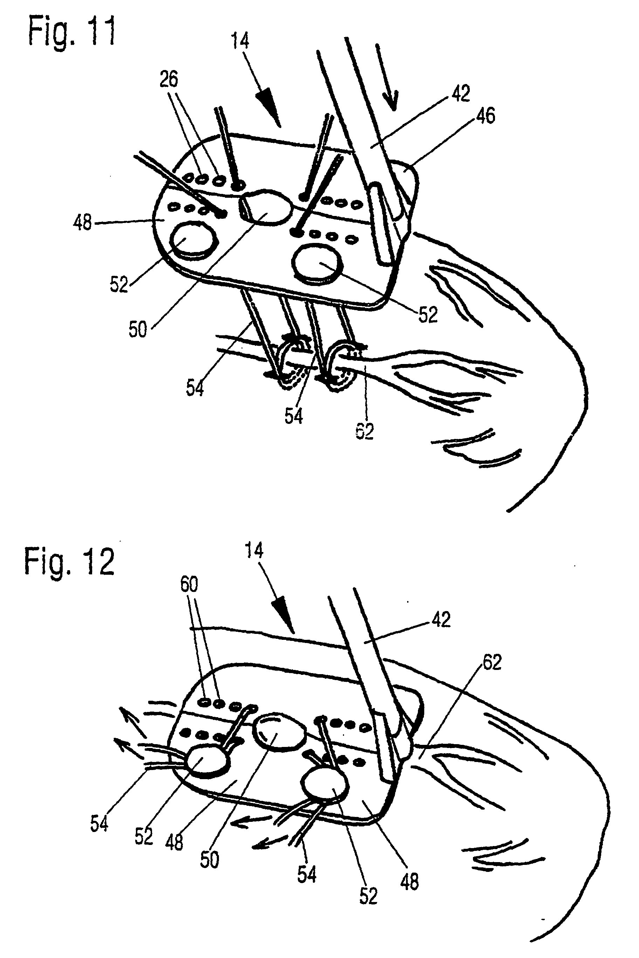

Auf wenigstens einem Plattformteilstück 48 sind Befestigungsmittel 52 ausgebildet, die zur Aufnahme und zum Belegen von Umschlingungsmitteln 54 dienen (vgl. Fig. 12).

Die Befestigungsmittel sind beispielsweise pilzartig ausgebildet, wie in Fig. 8 dargestellt, wobei ein Spalt 56 unter dem Pilzkopf 58 zur Aufnahme und zum Befestigen der Umschlingungsmittel dient. Dies ist beispielsweise aus den Fig. 6 und 12 ersichtlich.

Auf den Plattformteilstücken 46 und 48 sind ferner Öffnungen 60 ausgebildet, durch die die Umschlingungsmittel 54 durchführbar sind, wie in Fig. 11 bis 13 dargestellt. Die Öffnungen 60 dienen dabei als Lagerpunkte für die Mittel 54, die beispielsweise Vesselloops 54 sind, so daß das umschlungene Koronargefäß 62, wie aus Fig. 9 bis 11 ersichtlich, im Durchbruch 50 eingespannt und fixiert werden kann.

Fig. 10 zeigt eine zweite vorteilhafte Ausführungsform 200 der Plattform, wobei die Plattformteilstücke an einem Hebelmechanismus 48 befestigt sind. Wird das Gestänge 45 nach oben gezogen, so klappen die Plattformteilstücke 46 und 48 auf ganzer Breite auseinander.

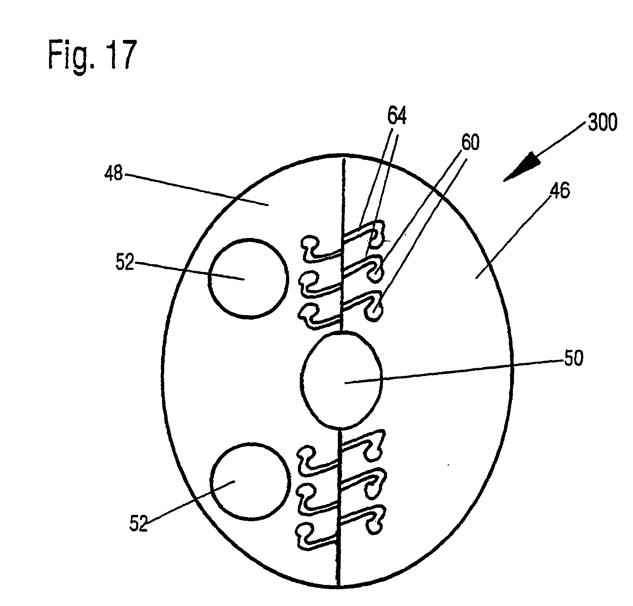

Fig. 17 zeigt eine dritte vorteilhafte Ausführungsform 300 der Plattform, wobei die Öffnungen 60 zusätzlich durch Schlitze 64 mit den Rändern der Plattformteilstücke 46 und 48 verbunden sind. Auf diese Weise kann ein mühsames Einfädeln der Vesselloops 54 in die Öffnungen entfallen. Die Vesselloops 54 werden statt dessen über die Schlitze 64 in den entsprechenden Öffnungen 60 bei geöffneten Plattformteilstücken 46 und 48 plaziert.

Hierbei sind die Schlitze 64 bevorzugt hakenförmig oder S-förmig ausgebildet, so daß ein unbeabsichtigtes Herausrutschen der Vesselloops 54 aus den Öffnungen 60 wirksam verhindert ist.

Handhabung, Funktionsweise und weitere Einzelheiten der erfindungsgemäßen Anordnung werden nachfolgend unter Bezugnahme auf Fig. 6, 11, 12, 13 und 16 erläutert.

Die Anordnung besteht bevorzugt aus chirurgischem Stahl und die Oberfläche ist durch Glasperlenstrahlen behandelt.

Die Säule 10 trägt den Ausleger 12 und stellt den Übergang zum jeweiligen Sperrer 16, 18, beispielsweise einem BIRNBAUM-Sperrer dar. Es ist bevorzugt, auswechselbare Adaptermodule oder einen Universaladapter vorzusehen, um die erfindungsgemäße Anordnung an alle im Gebrauch befindlichen Sperrer anschließen zu können.

In einer vorteilhaften Weiterbildung ist ein Kugelgelenk vorgesehen, das es ermöglicht, den Ausleger 12 in jeden sinnvollen Raumwinkel zu drehen, sowie ihn im Gelenk vor- und zurückschieben zu können. Eine solche Variation vereinfacht die Justierung der Plattform.

Die Konstruktion des Auslegergelenks 26 berücksichtigt bei seinen Abmessungen die Anforderungen an hohe Stabilität, freie Sicht, schnelle Aufnahme und Arretierung sowie ebenso schnelle Freigabe des Plattformstiels 42.

Die im Gelenk enthaltene Druckfeder 36 wird vorzugsweise vollständig von einer Hülse umschlossen, um im Fall eines Federbruchs das unkontrollierte Herabfallen von Bruchstücken in die Brusthöhle unmöglich zu machen.

Um den Reibungswiderstand des Herzens so gering wie möglich zu gestalten, ist die Unterseite der Plattformteilstücke 46 und 48 vorzugsweise poliert.

Die Ösen 60 für die Vesselloops 54 befinden sich,in vier Viereranordnungen rechts und links des ovalartigen Durchbruchs 50. Von großer Wichtigkeit für die Sicherheit ist das vollständige Entschärfen und Polieren dieser Ösen 60, damit ein Durchtrennen der Vesselloops 54 auf jeden Fall vermieden wird.

Zwei Knöpfe 52 dienen zur Arretierung der Vesselloops 54. Hierzu werden die Hohlschläuche oder hohle Gummizügel 54 um die Basis der Knöpfe 52 geschlungen und sind bereits nach einer Umdrehung fest arretiert.

Im Operationsbetrieb wird die Anordnung 100 ohne Plattform 14 angereicht, nachdem der LAD 62 vor und nach dem Anastomosenbereich mit Vesselloops 54 umschlungen wurde. Der Ausleger befindet sich in einer auf Erfahrung beruhenden mittleren Position, das Säulengelenk 22 ist geschlossen, das Auslegergelenk 26 geöffnet und parallel zur Säule 10 ausgerichtet. Die Flügelmuttern 24 und 28 weisen zum Assistenten.

Die Anordnung 100 ohne Plattform 14 wird auf die Basis des BIRNBAUM-Sperrers 16 aufgesetzt und in einer auf Erfahrung beruhenden mittleren Position durch Anziehen der Flügelschraube 20 an der Basis der Säule befestigt.

Durch abwechselndes Lösen und Schließen der Gelenke 22, 26 wird die Anordnung 100 so ausgerichtet, daß die Hülse des Auslegergelenkes 26 senkrecht auf den Anastomosenbereich weist, um ca. drei cm nach rechts versetzt.

Nun wird die Plattform 14 mit geschlossenen Plattformteilstücken 46, 48 angereicht und die Vesselloops 54 eingefädelt (Fig. 11).

Dann wird der Plattformstiel 42 in das geöffnete Auslegergelenk 26 eingelegt und das Gelenk 26 geschlossen, jedoch noch nicht festgesetzt.

Es folgt das gefühlvolle Anzüglen, Zentrieren und Absenken der Plattform 14 unter ständiger Abwägung der Faktoren "minimale Traumatisierung des LAD 62 durch die Vesselloops 54", "minimale Behinderung der Herzaktion", "optimale Ausrichtung der Plattform 14 (tangential zur Herzoberfläche, Anastomosenbereich zentriert, lange Achse des ovalartigen Durchbruchs 50 parallel zum LAD 62)," und schließlich "optimale Ruhigstellung des Anastomosenbereichs".

Wie in Fig. 12 dargestellt werden anschließend die Vesselloops 54 an den Knöpfen 52 festgesetzt. Nun kann der Operateur mit dem Nähen der Anastomose beginnen, wobei er ein optimal ruhiggestelltes und fixiert ausgerichtetes Koronargefäß 62 vor sich hat.

Nach fertiggestellter Anastomose werden die Plattformteilstücke 46 und 48 durch Betätigen der Rändelschraube 72 geöffnet, die Vesselloops gelöst und das Auslegergelenk 26 geöffnet, worauf sich die Plattform aus dem Operationsfeld entfernen läßt.

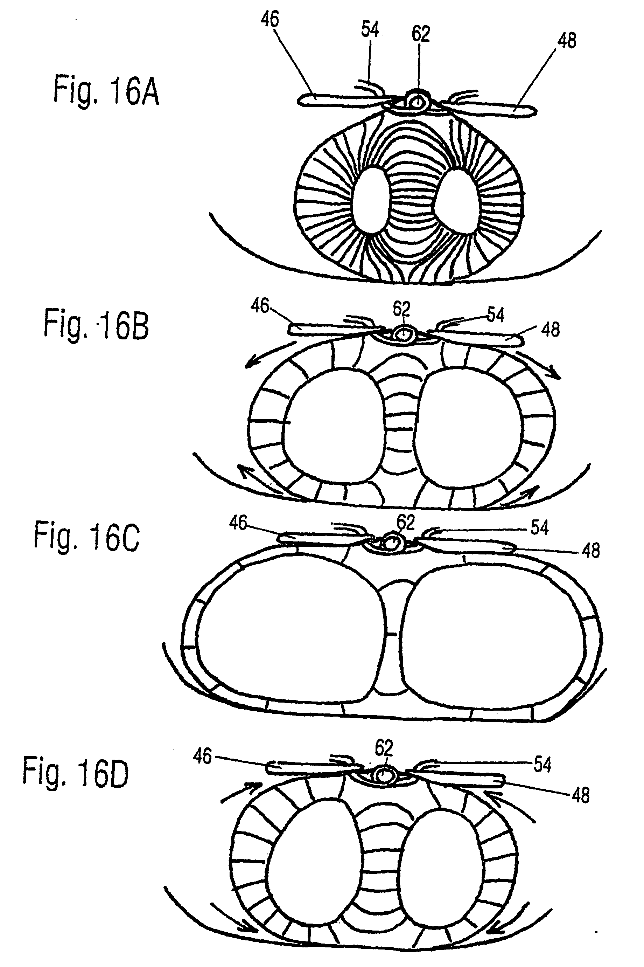

Fig. 16A bis 16D veranschaulichen die Situation bei fixiertem Koronargefäß 62 am schlagenden Herzen im kontraktierten Zustand (Fig. 16A), in der Expansionsphase (Fig. 16B), in expandiertem Zustand (Fig. 16C) und in der Kontraktionsphase (Fig. 16D). Hier wird deutlich, daß trotz unbehindert schlagendem Herzen das Koronargefäß 62 praktisch vollständig ruhiggestellt ist.

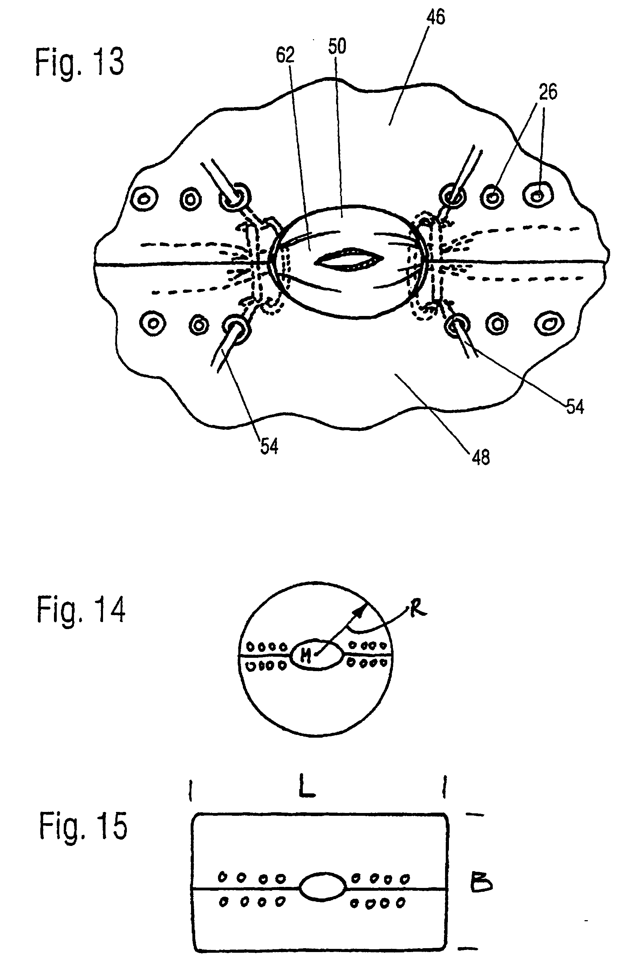

Fig. 13 zeigt im Detail das Anbringen der erfindungsgemäßen Anordnung am Herzen. Der LAD 62 wird rechts und links neben dem Anastomosenbereich von Vesselloops 54 umschlungen und diese in geeignete Ösen bzw. Öffnungen 60 der Plattform 14, 46, 48 gefädelt. Dann wird die Plattform abgesenkt, wobei die Vesselloops 54 leicht unter Spannung gehalten werden. Wenn die Plattform 14, 46, 48 aufgesetzt ist (vgl. Fig. 12), werden die Vesselloops 54 derart unter Spannung gesetzt, daß der LAD 62 verschlossen und der Anastomosenbereich im ovalartigen Durchbruch 50 zentriert ist.

Fig. 14 und 15 zeigen schematisch zwei Ausführungsformen der Plattformteilstücke als Anlageflächen. Bei der Ausführungsform gem. Fig. 15 ergänzen sich beide Plattformteilstücke 65, 66 zu einer Kreisfläche mit dem Radius R. Die so gebildete Auflagefläche beträgt bevorzugterweise zehn Quadratzentimeter.

Bei der Ausführungsform gemäß Fig. 16 bilden die beiden Plattformteilstücke 67, 68 ein Rechteck mit der Länge L und der Breite B. Die so gebildete Auflagefläche beträgt bevorzugterweise 30 Quadratzentimeter.

## Patentansprüche

1. Anordnung zum lokalen Ruhigstellen eines schlagenden Herzens, insbesondere zum Zweck der Herstellung einer Anastomose zwischen einem Bypassconduit und einem Koronargefäß (62) der Vorderwand der linken Herzkammer, mit einer Plattform (14), die aus mindestens zwei Plattformteilstücken besteht, welche so miteinander verbunden sind, daß sich die Plattformteilstücke (46, 48) so gegeneinander bewegen lassen, daß die Plattformteilstücke (46, 48) sich teilweise oder ganz voneinander lösen, **dadurch gekennzeichnet, daß** die Plattform (14) einen Durchbruch (50) aufweist, dessen Saum anteilig von beiden Plattformteilstücken (46, 48) gebildet wird.

2. Anordnung nach Anspruch 1, **dadurch gekennzeichnet, daß** der Durchbruch ovalartig ist.

3. Anordnung nach Anspruch 1 oder 2, **dadurch gekennzeichnet, daß** die Plattform in dem an den Durchbruch (50) angrenzenden Bereich mindestens eine Wölbung aufweist.

4. Anordnung nach einem der vorhergehenden Ansprüche, **dadurch gekennzeichnet, daß** auf den Plattformteilstücken (46, 48) wenigsten eine Öffnung (60) vorgesehen ist, wobei ferner Mittel (54) zum Umschlingen des Koronargefäßes (62) vorgesehen sind, welche durch die wenigstens zwei Öffnungen (60) hindurchführbar und an wenigstens einer auf den Gabelblättern (46, 48) vorgesehenen Befestigungsvorrichtungen (52) befestigbar sind.

5. Anordnung nach einem der voranstehenden Ansprüche, **dadurch gekennzeichnet, daß** der ovalartige Durchbruch einen kleinen Durchmesser von 7 mm bis 16 mm, insbesondere von 10 mm, und einen großen Durchmesser von 9 mm bis 20 mm, insbesondere von 14 mm hat.

6. Anordnung nach einem der vorhergehenden Ansprüche, **dadurch gekennzeichnet, daß** das Befestigungsmittel (52) wenigstens ein Profilknopf mit pilzartigem Querschnitt ist.

7. Anordnung nach einem der vorhergehenden Ansprüche, **dadurch gekennzeichnet, daß** die wenigsten zwei Öffnungen (60) je auf einem Plattformteilstück (46, 48) ausgebildet sind.

8. Anordnung nach einem der vorhergehenden Ansprüche **dadurch gekennzeichnet, daß** die wenigsten zwei Öffnungen (60) Bohrungen in den Plattformteilstücken (46, 48) sind.

9. Anordnung nach einem der vorhergehenden Ansprüche, **dadurch gekennzeichnet, daß** wenigstens ein Plattformteilstück (46, 48) an einer am Herzen aufliegenden Seite zum Durchbruch (50) hin angefast ist.

10. Anordnung nach einem der vorhergehenden Ansprüche, **dadurch gekennzeichnet, daß** das Mittel (54) zum Umschlingen des Koronargefäßes (62) wenigstens ein Faden oder wenigstens ein Vesselloop ist.

11. Anordnung nach einem der vorhergehenden Ansprüche, **dadurch gekennzeichnet, daß** in den Plattformteilstücken (46, 48) Schlitze (64) vorgesehen sind, welche ausgehend von den Kanten, an denen sich die Plattformteilstücke (46, 48) berühren, zu je einer Öffnung (60) verlaufen.

12. Anordnung nach Anspruch 11, **dadurch gekennzeichnet, daß** die Schlitze (64) jeweils haken- oder S-förmig ausgebildet sind.

## Claims

1. An arrangement for locally immobilising a beating heart, in particular for the purpose of producing an anastomosis between a bypass conduit and a coronary vessel (62) of the front wall of the left ventricle, comprising a platform (14) consisting of at least two platform parts connected to one another in such a way that the platform parts (46, 48) can be moved relative to one another so as to be partially or completely separated, **characterised in that** the platform (14) has a gap (50), the edge of which is proportionally formed by the two platform parts (46, 48).

2. An arrangement according to claim 1, **characterised in that** the gap is oval-shaped.

3. An arrangement according to claim 1 or 2, **characterised in that** the platform has at least one convexity in the region adjacent to the gap (50).

4. An arrangement according to any one of the preceding claims, **characterised in that** at least one opening (60) is provided in the platform parts (46, 48), wherein means (54) for looping around the coronary vessel (62) are also provided which are insertable through the at least two openings (60) and are fixable to at least one fixing device (52) provided on the fork plates (46, 48).

5. An arrangement according to any one of the preceding claims, **characterised in that** the oval-shaped gap has a minor diameter of 7 mm to 16 mm, in particular 10 mm, and a major diameter of 9 mm to 20 mm, in particular 14 mm.

6. An arrangement according to any one of the preceding claims, **characterised in that** the fixing means (52) is at least one profiled stud with a mushroom-shaped cross-section.

7. An arrangement according to any one of the preceding claims, **characterised in that** the at least two openings (60) are formed in each platform part (46, 48).

8. An arrangement according to any one of the preceding claims, **characterised in that** the at least two openings (60) are bores in the platform parts (46, 48).

9. An arrangement according to any one of the preceding claims, **characterised in that** at least one platform part (46, 48) is chamfered in the direction of the gap (50) on the side resting against the heart.

10. An arrangement according to any one of the preceding claims, **characterised in that** the means (54) for looping around the coronary vessel (62) is at least one thread or at least one vessel loop.

11. An arrangement according to any one of the preceding claims, **characterised in that** slots (64) are provided in the platform parts (46, 48) and, starting from the edges along which the platform parts (46, 48) contact one another, each extend to an opening (60).

12. An arrangement according to claim 11, **characterised in that** the slots (64) are each hook-shaped or S-shaped.

## Revendications

1. Agencement pour calmer localement un coeur en cours de battements, en particulier dans le but d'établir une anastomose entre un conduit de by-pass et un vaisseau coronaire (62) de la paroi antérieure du ventricule gauche, comprenant une plate-forme (14) qui est constitué par au moins deux parties de plate-forme, lesquelles sont reliées l'une à l'autre de telle façon que les parties de plate-forme (46, 48) peuvent être déplacées l'une par rapport lors à l'autre de telle manière que les parties de plate-forme (46, 48) se détachent partiellement ou totalement l'une de l'autre, **caractérisé en ce que** la plate-forme (14) présente une traversée (50), dont l'ourlet est constitué à proportion par les deux parties de plate-forme (46,48).

2. Agencement selon la revendication 1, **caractérisé en ce que** la traversée est de forme ovale.

3. Agencement selon l'une ou l'autre des revendications 1 et 2, **caractérisé en ce que** la plate-forme présente au moins un bombement dans la région adjacente à la traversée (50).

4. Agencement selon l'une des revendications précédentes, **caractérisé en ce qu'**il est prévu au moins une ouverture (60) sur les parties de plate-forme (46, 48), et **en ce qu'**il est prévu en outre des moyens (54) pour entourer le vaisseau coronaire (62), ces moyens pouvant être passés à travers lesdites au moins deux ouvertures (60), et pouvant être fixés sur au moins un dispositif de fixation (52) prévu sur les parties de fourches (46, 48).

5. Agencement selon l'une des revendications précédentes, **caractérisé en ce que** la traversée de forme ovale possède un petit diamètre de 7 à 16 mm, en particulier de 10 mm, et un grand diamètre de 9 à 20 mm, en particulier de 14 mm.

6. Agencement selon l'une des revendications précédentes, **caractérisé en ce que** le moyen de fixation (52) est au moins un bouton profilé à section en forme de champignon.

7. Agencement selon l'une des revendications précédentes, **caractérisé en ce que** lesdites au moins deux ouvertures (60) sont ménagées chacune sur une partie de plate-forme (46, 48).

8. Agencement selon l'une des revendications précédentes, **caractérisé en ce que** lesdites au moins deux ouvertures (60) sont des perçages dans les parties de plate-forme (46,48).

9. Agencement selon l'une des revendications précédentes, **caractérisé en ce qu'**une partie de plate-forme (46, 48) au moins est chanfreinée sur un côté tourné vers la traversée (50) et appliqué contre le coeur.

10. Agencement selon l'une des revendications précédentes, **caractérisé en ce que** les moyens (54) pour entourer le vaisseau coronaire (62) est au moins un fil, ou au moins une boucle de Vessel.

11. Agencement selon l'une des revendications précédentes, **caractérisé en ce que** des fentes (64) sont prévues dans les parties de plate-forme (42, 48), lesdites fentes partant des bords au niveau desquels les parties de plate-forme (46, 48) se touchent, en s'étendant jusqu'à une ouverture respective.

12. Agencement selon la revendication 11, **caractérisé en ce que** les fentes (64) sont respectivement réalisées sous forme de crochet ou sous forme de S.
